# FASCICULE DE BREVET EUROPEEN

(11) **EP 0 482 976 B1**
(45) Date de publication et mention de la délivrance du brevet: **13.12.1995**
(21) Numéro de dépôt: 91402660.4
(22) Date de dépôt: 04.10.1991
(51) Int. Cl.: A61M 5/31, A61F 2/02

(54) **Seringue d'injection**
Injektionsspritze
Injection syringe

(30) Priorité: 23.10.1990 FR 9013105
(43) Date de publication de la demande: 29.04.1992
(73) Titulaire: B. BRAUN CELSA, 86360 Chasseneuil du Poitou (FR)
(72) Inventeur: Nadal, Guy, F-86000 Poitiers (FR)
(74) Mandataire: Lerner, François

(56) Documents cités:
- DE-A- 1 491 768
- FR-A- 973 467
- FR-A- 2 625 672

## Description

L'invention concerne une seringue d'introduction d'un produit, tel en particulier qu'une prothèse vasculaire, vers le corps d'un receveur.

Dans le domaine médical, l'utilisation de seringues est bien entendu largement répandue, ces seringues comprenant, comme connu en soi, un corps de seringue formant cylindre pour une tige de piston destinée à se déplacer dans ce corps pour pousser le produit qui y est contenu de manière à l'introduire vers l'endroit approprié du corps du patient receveur.

Parmi les nombreuses seringues existantes, certaines sont utilisées pour la mise en place de filtres sanguins conformés pour être à la fois perméables au flux de sang, tout en arrêtant les caillots susceptibles de migrer vers le coeur, afin d'éviter en particulier les risques d'embolie. Les filtres les plus connus se présentent souvent sous la forme d'un petit panier tronconique comportant une série de pattes filiformes. On en trouve des exemples en particulier dans les publications FR-A-2 570 288 et FR-A-2 573 646, ainsi que dans US-A-3 952 747.

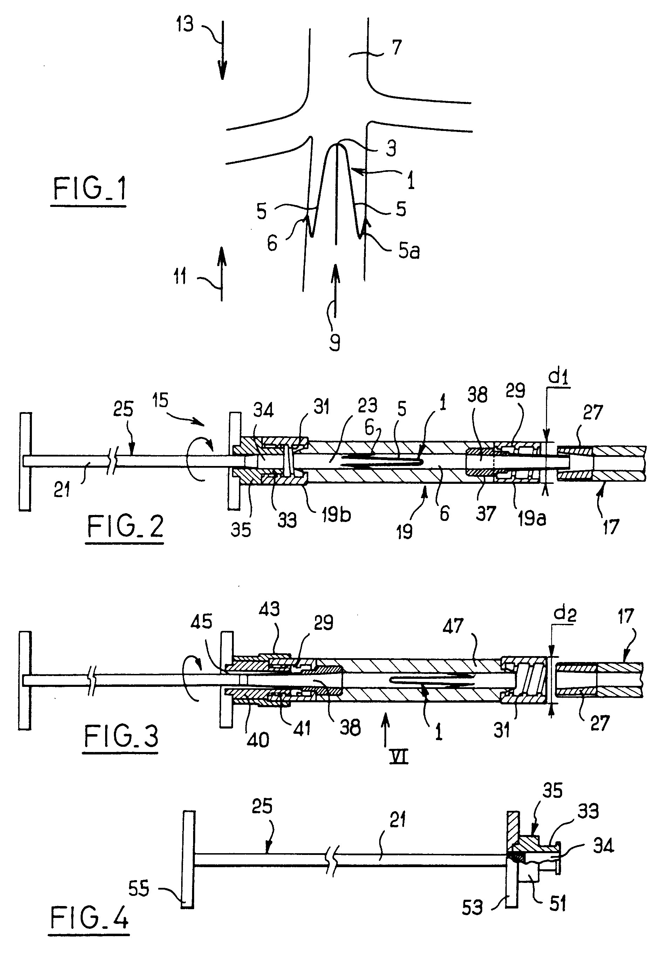

De tels filtres peuvent être posés par voie jugulaire ou par voie fémorale. Mais dans les deux cas, le filtre doit toujours être disposé avec la même orientation dans la veine, comme cela est illustré sur la figure 1 où l'on a repéré en 1 un tel filtre sanguin. Celui-ci se présente sous la forme d'une sorte de panier présentant une tête sensiblement ogivale 3 de laquelle partent des bras ou pattes 5 généralement expansibles et éventuellement pourvu(e)s de crochets 6 d'ancrage à la paroi de la veine. Les pattes 5 vont en s'évasant de manière à venir porter, par leur extrémité libre 5a, contre la paroi de la veine 7 à l'intérieur de laquelle le filtre est sensiblement centré en étant orienté de manière que le flux sanguin (dont le sens a été schématisé par la flèche 9) rencontre d'abord les pattes puis la tête de jonction plus étroite 3.

Sur cette figure 1, on a également repéré par les flèches 11 et 13 respectivement le sens de mise en place du filtre par voie fémorale (flèche 11) et par voie jugulaire (repère 13). A partir de cette illustration, on comprendra qu'il convient impérativement d'orienter correctement le filtre lors de sa mise en place dans le matériel de pose (en l'espèce la seringue) afin que ce filtre puisse être délivré le moment voulu en présentant en premier soit sa tête 3 si la voie d'accès fémorale a été choisie, soit ses pattes 5 si l'on a privilégié la voie jugulaire.

Il existe actuellement deux techniques principales de pose sans et avec préconditionnement de la prothèse à introduire.

Dans le premier cas, le praticien oriente manuellement la prothèse au moment de son introduction dans le matériel de pose. Une erreur humaine est alors toujours possible. De plus, la prothèse risque d'être détériorée lors de sa manipulation.

Lorsque le filtre est préconditionné, il est alors contenu dans une seringue dont l'extrémité libre, (opposée à celle portant la tige de piston) est prévue pour venir se connecter sur le matériel de pose, tel qu'un cathéter, à travers lequel la prothèse va circuler jusqu'à son endroit de libération.

Dans ce cas, le chirurgien doit impérativement choisir, avant l'intervention, la voie d'abord du patient et ne peut plus ensuite en changer. Or, il arrive que le chirurgien soit amené à reconsidérer son choix.

Avec ce système, il ne peut passer d'une voie d'abord à l'autre qu'en venant à nouveau installer sur l'embout du cathéter un nouvel ensemble préconditionné comprenant une nouvelle seringue complète renfermant une nouvelle prothèse placée dans le sens correspondant à la voie d'abord maintenant choisi. Ceci impose donc d'avoir à disposition un double stock de seringues préconditionnées. En outre, la perte de temps que représente le changement d'une seringue complète par une autre multiplie les risques opératoires.

Dans le domaine des seringues, on connaît notamment les réalisations décrites dans DE 1 491 768 et FR 973467.

Dans FR 973467, il s'agit d'une seringue démontable, ce qui facilite son nettoyage et sa stérilisation, le dispositif décrit comprenant :
- un corps de seringue dans lequel peut coulisser une tige de piston,
- un piston avec sa dite tige,
- et des moyens d'assemblage pour, à une première extrémité du corps de seringue, autoriser un assemblage amovible du piston audit corps, et, à une seconde extrémité opposée à la première, autoriser le raccordement amovible de ce même corps avec un moyen complémentaire d'introduction du contenu de la seringue vers le corps du patient.

Mais cette seringue ne propose pas de solution susceptible de résoudre les problèmes évoqués ci-avant du praticien.

Par ailleurs, dans DE 1 491 768, on trouve une seringue dont la fabrication paraît facilitée, évitant les problèmes soulevés notamment d'alignement entre le piston et le corps de la seringue. Mais l'on ne trouve, là encore, aucune solution aux problèmes de l'invention.

La seringue perfectionnée proposée dans la présente invention a donc tout particulièrement pour objet de remédier aux problèmes rencontrés sur les systèmes existants.

En bref, la solution proposée consiste à utiliser un corps de seringue ouvert à ses deux extrémités opposées et relié, à l'une d'elles, à un piston d'actionnement, des moyens de détrompage de sécurité prévus sur le piston et sur le corps de seringue évitant toute introduction "à l'envers".

Plus précisément, le dispositif de l'invention, qui est donc du type comprenant un corps de seringue, un piston et des moyens d'assemblage amovible corps/piston, comme indiqué ci-dessus en relation avec FR 973467, se caractérise en ce qu'il comprend en outre :
- un second piston, les moyens d'assemblage entre le premier piston et le corps de seringue n'étant compatibles en assemblage entre eux qu'à ladite première extrémité du corps, de même pour le second piston à la seconde extrémité de ce même corps où les moyens d'assemblage entre ledit corps et le second piston sont conformés en conséquence, les moyens d'assemblage formés à ladite première extrémité du corps étant en outre compatibles avec le raccordement amovible audit moyen complémentaire, hors la présence dudit premier piston.

Ces moyens de sécurité prévus donc sur le corps et les pistons imposeront qu'un type donné de piston ne pourra venir coopérer qu'avec l'une des extrémités du corps de seringue. On conseille au demeurant de conformer différemment les deux moyens de jonction prévus aux deux extrémités opposées du corps de la seringue, ce corps pouvant présenter, à côté des moyens de jonction en question, un profil ou une forme spécifique à chaque extrémité.

Il a également été envisagé que la seringue puisse comprendre des éléments visuels extérieurs de repérage comportant un code de couleurs commun à une partie d'un piston donné et à un endroit approprié du corps de la seringue correspondante, de manière à inciter l'utilisateur à mettre en place le piston choisi d'un côté donné du corps de cette seringue.

Selon une caractéristique complémentaire chaque piston pourra en outre comprendre une bague traversée par la tige de piston et pourvue de premiers moyens d'assemblage propres à coopérer avec des seconds moyens d'assemblage complémentaires du corps de seringue, pour constituer lesdits moyens d'assemblage de l'un des pistons étant différents de ceux de l'autre piston.

Grâce aux caractéristiques de l'invention, le praticien sera donc en mesure de réagir très rapidement s'il s'avère que la voie d'abord choisie est inappropriée, ayant à sa disposition les moyens lui permettant de changer, sans risque, de voie d'abord.

A la lecture de ce qui précède, on aura compris qu'une partie de l'invention a résidé dans la mise au point d'un piston pour corps de seringue, prévu en particulier pour une seringue du type mentionné ci-avant, se caractérisant en ce qu'aux moyens d'assemblage dont il est équipé est associé un profil de détrompage tel que le piston ne puisse coopérer avec les moyens d'assemblage complémentaires du corps de seringue qu'à l'une des deux extrémités de ce corps.

D'autres caractéristiques et avantages de l'invention apparaîtront encore de la description qui va suivre faite en référence aux dessins annexés dans lesquels :
- outre la figure 1 déjà présentée qui illustre un exemple de filtre sanguin en place sur un trajet veineux,
- les figures 2 et 3 montrent, en vue de coupe longitudinale, un même corps de seringue pourvu a l'une ou l'autre ou l'autre de ses extrémités d'un piston adapté,
- les figures 4 et 5 présentent en coupe partielle et isolément les pistons utilisés sur le corps de la seringue des figures 2 et 3, respectivement,
- les figures 6 et 7 montrent en vue extérieure de côté, dans le sens des flèches VI et VII des figures 2 et 3, le corps de seringue seul portant des éléments d'identification visuels permettant de repérer le sens d'introduction prévu du produit ou élément contenu dans le corps de cette seringue,
- et la figure 8 illustre en vue perspective une variante de réalisation de la seringue de l'invention.

Si l'on se reporte tout d'abord aux figures 1 et 2, on voit donc représenté une seringue destinée en l'espèce à assurer l'introduction, via un cathéter 17, de la prothèse 1 en place pour l'instant à l'intérieur du corps 19 de la seringue. Ce corps se présente comme une pièce tubulaire ouverte à ses deux extrémités opposées 19a, 19b de façon à permettre l'engagement (dans la seringue) ou l'expulsion, (notamment vers le cathéter 17), de l'élément 1. Ces ouvertures sont également conformées pour permettre le montage d'un piston dont on aperçoit en 21 la tige d'actionnement qui est bien entendu adaptée pour pénétrer à l'intérieur du passage 23 du corps de la seringue où est ici conditionnée la prothèse.

Du côté de sa première extrémité d'ouverture 19a, le corps de seringue est équipé d'un premier moyen de jonction constitué en l'espèce d'un embout taraudé 29 adapté pour venir coopérer de façon amovible avec les filets d'un embout complémentaire 27 prévu sur le cathéter 17. A son extrémité opposée 19b, ce même corps de seringue comprend un autre embout (ou bague) également équipé(e) d'un moyen de jonction constitué en l'espèce également par une partie ou une bague taraudée à l'intérieur de laquelle sont ici engagés (de façon amovible) les filets complémentaires de jonction 33 de la bague 35 qui, avec la tige 21, constitue le piston 25. Bien entendu, le passage intérieur 34 réservé, au milieu de la bague 35, à la tige 21 a été adapté pour que les passages 34 et 23 puissent coïncider et assurent à la tige 21 le meilleur guidage en translation.

Dans la mesure où, dans l'exemple illustré, la seringue retenue est destinée à assurer notamment l'introduction d'un filtre sanguin percutané pourvu de crochets 6 risquant de venir s'agripper à la paroi de l'embout 27 du cathéter, on remarquera que l'extrémité de droite sur la figure 2 (et de gauche sur la figure 3) du corps de la seringue est pourvue d'un fin embout complémentaire 37 tronconique extérieurement et traversé de part en part par un canal 38 de même diamètre que le passage 23 dans la continuité duquel il est formé. La forme et la longueur de l'embout 37 ont été ici adaptées pour qu'il passe à travers la partie taraudée 29 et puisse déboucher dans le cathéter 17, permettant ainsi aux crochets 6 du filtre de passer sans encombre le cap souvent critique de l'embout 27, sa forme effilée lui permettant par ailleurs de n'entraver en rien la liaison entre le cathéter et les moyens de jonction associés du corps de la seringue.

En fait, dans le cas présent, cet embout 37 pourra également servir de moyen de repérage du sens d'introduction du filtre puisqu'il n'est prévu qu'à l'une des extrémités du corps de seringue.

Mais cet embout ne jouera de préférence qu'un rôle complémentaire de repérage, la sécurité liée au sens de déplacement du filtre étant de préférence essentiellement assurée par la présence de deux pistons différents, ou plus exactement de deux bagues de piston différentes pourvues chacune de moyens de jonction spécifiques adaptables uniquement sur l'un ou l'autre des deux embouts de jonction 29, 31 du corps de la seringue.

Cela est d'ailleurs clairement illustré si l'on compare les figures 2 et 3, la figure 3 montrant une bague de piston 40 qui se différencie de celle de la figure 2 dans la mesure où, à sa partie filetée 41 qui est adaptée pour venir coopérer par l'intérieur avec le taraudage 29 du même corps 19 de seringue, est associée une partie annulaire extérieur 43 venant entourer localement l'embout 29. En donnant par exemple des profils ou des diamètres extérieurs d₁ et d₂ différents aux embouts 29 et 31, on interdira ainsi à la bague de montage 40 de venir coopérer avec l'embout d'extrémité 31 "non autorisé" du corps de la seringue. En outre, avec une telle structure il sera possible de conserver aux deux extrémités 29 et 31 un taraudage identique, permettant ainsi l'utilisation d'un même cathéter 17, que son embout 27 vienne se connecter à l'une ou l'autre des extrémités du corps de seringue pour un engagement dans un sens ou dans l'autre du filtre.

Mais on aurait bien entendu, à titre de variante, pu prévoir des embouts 29 et 31 équipés de moyens de jonction différents l'un de l'autre (chacun de ces moyens restant toutefois bien entendu compatible avec l'une ou l'autre des bagues de montage du piston).

Toujours sur la figure 3, on remarquera encore la bague de jonction 40 présente ici un passage intérieur 45 légèrement plus large que celui permettant un guidage étroit de la tige 21 du piston, de telle sorte que cette tige est essentiellement guidée dès l'entrée du corps de la seringue par la paroi du canal 38 de la garniture 37.

Pour compléter la description du corps de seringue, on remarquera également, et cela plus clairement sur la figure 3, que ce corps pourra par exemple être réalisé de manière que ses embouts de jonction 29, 31 soient reliées entre-eux par une partie tubulaire intermédiaire 47 s'arrêtant, du côté opposé à la garniture 37, juste à l'entrée de l'embout 31, aucune prolongation n'étant ici nécessaire dans la mesure où, dans cette configuration, le filtre doit sortir de la seringue pattes en avant.

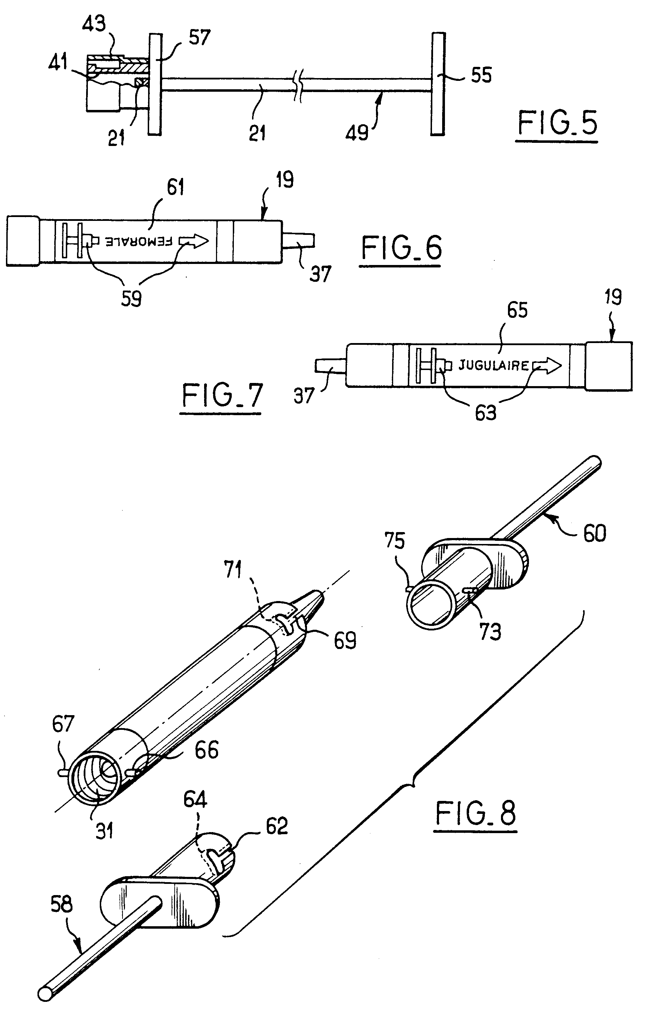

Reportons-nous maintenant aux figures 4 et 5 pour voir illustré seuls les deux types de pistons qui ont a priori été privilégiés.

Dans le cadre de l'utilisation du concept de l'invention, il a été choisi arbitrairement que le piston repéré 25 sur la figure 4 (et que l'on retrouve sur la figure 2) soit utilisé lorsque l'on désirera introduire le filtre par voie fémorale, c'est-à-dire, lorsque la seringue devra être préparée comme illustrée sur la figure 2.

Toujours sur la figure 4 on remarquera la forme de la bague 35 qui porte les moyens de jonction du piston et à travers le passage 34 de laquelle peut bien entendu glisser la tige 21. En arrière de sa partie fileté 33, la bague 35 présente un épaulement extérieur 51 formant butée d'arrêt, puis une collerette d'appui 53, tandis que la tige 21 se termine du côté opposé à celui par lequel elle s'engage à travers le passage 34 de la bague par une partie élargie formant poussoir 55.

Si l'on regarde maintenant l'autre piston de la figure 5, on peut remarquer qu'il présente également, à l'arrière de sa partie intérieure filetée 41 qu'entoure le profil annulaire 43, une collerette d'appui 57, la même tige 21 avec son poussoir 55 pouvant être utilisée pour l'un et l'autre des pistons.

Pour compléter l'identification de l'orientation correcte de la seringue, il a été envisagé de lui associer des éléments visuels extérieurs de repérage pouvant comporter un code de couleurs commun à une partie du piston et du corps de seringue.

Dans cet esprit, on pourra par exemple prévoir de réaliser en une couleur bleue la collerette et le poussoir 55 du piston 25 (destiné dans l'exemple choisi à une introduction par voie fémorale), tandis qu'une couleur rouge pourait être donnée aux mêmes pièces 55 et 57 du piston 49 (choisi dans cet exemple pour une introduction par voie jugulaire). Quant au corps de seringue, il pourra par exemple comporter d'un côté (voir figure 6) des repères 59 et une mention 61 indiquant par exemple, pour la voie d'accès fémorale, l'extrémité où doit être installé le piston et l'extrémité par laquelle doit sortir le filtre, tandis que du côté opposé (voir figure 7), ce même corps pourra porter extérieurement des marques 63 et 65 indiquant les mêmes repères inversés pour une introduction par voie jugulaire. De plus, les mêmes couleurs que celles utilisées pour les pistons pourraient être retenues pour les mentions portées d'un côté ou de l'autre du corps de seringue, suivant que l'une ou l'autre des voies d'accès a été retenue.

Bien que le principe d'une jonction amovible piston/corps de seringue du type pas de vis male/femelle puisse être préféré , on pourrait bien entendu envisager d'autres types de jonction mécanique susceptibles d'éviter les risques d'erreurs dans le sens d'introduction du filtre, tels que par exemple les jonctions du type à baïonnette ou à formes géométriques associées carrées, hexagonales...

Sur la figure 8, on a à titre d'exemple illustré une variante de réalisation de la seringue avec une association piston/corps de seringue du type à baïonnette.

Dans cet exemple, la bague de jonction du premier piston repéré 58 est équipée de deux fentes en "T" 62, 64 adaptées pour venir coopérer avec deux tétons 66, 67 prévus de façon complémentaire à l'une des extrémités du corps de seringue associé, ce corps présentant lui-même à son extrémité opposée deux fentes en "T" 69, 71 diamétralement opposées adaptées pour recevoir les deux tétons associés 73, 75 prévus bien entendu de façon complémentaire sur l'embout creux ou bague de l'autre piston 60.

Il est clair qu'un tel mode de liaison n'interdit aucunement au corps de la seringue de présenter intérieurement, à ses extrémités, les mêmes taraudages (tels que 31) destinés à assurer la liaison avec le cathéter 17 complémentaire d'introduction, reprenant en cela les avantages offerts par les seringues des figures 2 et 3.

Dans une éventuelle application complémentaire, on notera qu'à titre de variante de réalisation on pourrait imaginer de rendre solidaire les embouts 29, 31 du corps de seringue et les bagues percées correspondantes 40, 35 des pistons (seules les tiges de piston étant alors amovibles), quitte à prévoir par exemple un taraudage du côté de l'extrémité extérieure de ces bagues pour permettre si nécessaire une liaison avec un cathéter du type par exemple de celui repéré 17 sur la figure 2.

Bien entendu, même si la description qui précède a été faite essentiellement dans le cadre d'une seringue destinée à l'introduction d'un filtre sanguin, on pourrait prévoir d'utiliser cette seringue (en adaptant ses dimensions et notamment celles des orifices et passages) pour injecter d'autres produits tels que d'autres types de prothèse, voire des liquides.

## Revendications

1. Dispositif pour introduire le contenu d'un corps (19) de seringue vers le corps d'un patient, comprenant :
- ledit corps (19) de seringue dans lequel peut coulisser une tige (21) de piston,
- un piston (25, 58) avec sa dite tige (21),
- et des moyens d'assemblage (29, 31, 33, 41 ; 66, 69, 62, 73) pour, à une première extrémité du corps (19) de seringue, autoriser un assemblage amovible du piston audit corps, et, à une seconde extrémité opposée à la première, autoriser le raccordement amovible de ce même corps avec un moyen complémentaire (17) d'introduction du contenu de la seringue vers le corps du patient, caractérisé en ce qu'il comprend en outre :
- un second piston (49, 60), les moyens d'assemblage entre le premier piston (25, 58) et le corps (19) de seringue n'étant compatibles en assemblage entre eux qu'à ladite première extrémité du corps, de même pour le second piston (49, 60) à la seconde extrémité de ce même corps où les moyens d'assemblage entre ledit corps et le second piston sont conformés en conséquence, les moyens d'assemblage formés à ladite première extrémité du corps étant en outre compatibles avec un raccordement amovible audit moyen complémentaire (17), hors la présence dudit premier piston.

2. Dispositif selon la revendication 1 caractérisé en ce que chaque piston (25, 49, 58, 60) comprend une bague (35, 40) traversée par la tige (21) de piston et pourvue de premiers moyens d'assemblage (33, 41) propres à coopérer avec des seconds moyens d'assemblage complémentaires (29, 31) du corps de seringue, pour constituer lesdits moyens d'assemblage précités, lesdits premiers moyens d'assemblage de l'un des pistons étant différents de ceux de l'autre piston.

3. Dispositif selon la revendication 1 ou la revendication 2 caractérisé en ce que le corps (19) de seringue est, à proximité desdits moyens d'assemblage (29, 31) qu'il porte, conformé différemment à l'une et l'autre de ses deux extrémités.

4. Dispositif selon la revendication 3 caractérisé en ce que le corps (19) de seringue comporte à chacune de ses deux extrémités opposées (19a, 19b) un embout (29, 31) portant lesdits moyens d'assemblage du corps, ce corps comportant à l'une seulement de ses dites deux extrémités une partie tubulaire tronconique (37) traversant l'un des embouts pour n'autoriser l'assemblage, à cette extrémité du corps, qu'avec le piston (25, 49 ; 58, 60) correspondant.

5. Dispositif selon l'une quelconque des revendications 2 à 4 caractérisé en ce que lesdits seconds moyens d'assemblage (29, 31) prévus sur le corps (19) de seringue, à chacune de ses extrémités, sont conformés pour pouvoir également constituer moyens de raccordement amovibles avec ledit moyen (17) complémentaire d'introduction de son contenu vers le corps du patient.

6. Dispositif selon l'une quelconque des revendications précédentes caractérisé en ce que les moyens d'assemblage (29, 31) du corps de seringue consistent en des taraudages pour un verrouillage par vissage avec l'un ou l'autre desdits pistons (35, 40 ; 58, 60) et/ou avec le moyen complémentaire (17) d'introduction du contenu de la seringue vers le corps du patient.

7. Dispositif selon l'une quelconque des revendications précédentes caractérisé en ce que les pistons (25, 49 ; 58, 60) et le corps (19) de seringue comportent des éléments visuels extérieurs (59, 61, 63, 65, 53, 55, 57) de repérage du côté d'assemblage prévu du piston correspondant sur ce corps de seringue.

8. Dispositif selon la revendication 7 caractérisé en ce que lesdits éléments visuels extérieurs comportent un code de couleur différent pour chaque piston et commun à une partie du piston correspondant et du corps de seringue.

9. Dispositif selon l'une quelconque des revendications précédentes caractérisé en ce qu'à l'une des extrémités du corps (19) de seringue, les moyens d'assemblage (29 ; 66, 67) de ce dernier coopèrent avec ceux du premier piston (49, 58) par recouvrement partiel de ladite première extrémité du corps par une partie dudit premier piston, tandis qu'à la seconde extrémité, le second piston (25, 60) et le corps coopèrent en assemblage uniquement par engagement partiel de ce piston dans cette extrémité du corps de seringue.

10. Dispositif selon l'une quelconque des revendications précédentes, caractérisé en ce que le contenu du corps (19) de seringue est un filtre sanguin (1) implantable dans un vaisseau pour y arrêter des caillots de sang.

## Claims

1. Device for introducing the contents of a syringe element (19) towards the body of a patient, comprising:
- the said syringe element (19) in which a plunger rod (21) can slide,
- a plunger (25, 58) with its said rod (21),
- assembly means (29, 31, 33, 41; 66, 69, 62, 73) for permitting, at a first end of the syringe element (19), a removable assembly of the plunger to the said element and, at a second end opposite the first one, permitting the removable connection of this same element to a complementary means (17) for introduction of the contents of the syringe towards the body of the patient, characterised in that it additionally comprises;
- a second plunger (49, 60), the assembly means between the first plunger (25, 58) and the syringe element (19) being compatible for assembling to each other only at the said first end of the element, and similarly as regards the second plunger (49, 60) at the second end of this same element where the means for assembly between the said element and the second plunger are designed accordingly, the assembly means formed at the said first end of the element moreover being compatible with a removable connection to the said complementary means (17), in the absence of the said first plunger.

2. Device according to Claim 1, characterised in that each plunger (25, 49, 58, 60) comprises a ring (35, 40) passed through by the plunger rod (21) and provided with first assembly means (33, 41) capable of cooperating with second complementary assembly means (29, 31) of the syringe element, in order to constitute the said above-mentioned assembly means, the said first assembly means of one of the plungers being different from those of the other plunger.

3. Device according to Claim 1 or Claim 2, characterised in that the syringe element (19) is, in the proximity of the said assembly means (29, 31) which it bears, designed differently at each of its two ends.

4. Device according to Claim 3, characterised in that the syringe element (19) comprises at each of its two opposite ends (19a, 19b) an endpiece (29, 31) bearing the said assembly means of the element, this element comprising at only one of its said two ends a truncated tubular part (37) passing through one of the endpieces in order to permit assembly, at this end of the element, only with the corresponding plunger (25, 49; 58, 60).

5. Device according to any one of Claims 2 to 4, characterised in that the said second assembly means (29, 31) provided on the syringe element (19), at each of its ends, are designed so as to be able also to constitute means for removable connection to the said complementary means (17) of introduction of its contents towards the body of the patient.

6. Device according to any one of the preceding claims, characterised in that the assembly means (29, 31) of the syringe element consist of tappings for a screw-type locking with one or other of the said plungers (35, 40; 58, 60) and/or with the complementary means (17) for introduction of the contents of the syringe towards the body of the patient.

7. Device according to any one of the preceding claims, characterised in that the plungers (25, 49; 58, 60) and the syringe element (19) comprise external visual elements (59, 61, 63, 65, 53, 55, 57) for indicating the direction of intended assembly of the corresponding plunger on this syringe element.

8. Device according to Claim 7, characterised in that the said external visual elements comprise a colour code which is different for each plunger and which is common to one part of the corresponding plunger and of the syringe element.

9. Device according to any one of the preceding claims, characterised in that, at one of the ends of the syringe element (19), the assembly means (29; 66, 67) of the latter cooperate with those of the first plunger (49, 58) by partial covering of the said first end of the element by a part of the said first plunger, while, at the second end, the second plunger (25, 60) and the element cooperate solely by partial engagement of this plunger in this end of the syringe element.

10. Device according to any one of the preceding claims, characterised in that the contents of the syringe element (19) is a blood filter (1) which can be implanted in a vessel in order to stop blood clots therein.

## Patentansprüche

1. Vorrichtung zum Einführen des Inhaltes eines Spritzenkörpers (19) zum Körper eines Patienten hin, mit:
- dem Spritzenkörper (19), in welchem eine Kolbenstange (21) gleiten kann,
- einem Kolben (25, 58) mit seiner Stange (21),
- und Mitteln für den Zusammenbau (29, 31, 33, 41; 66, 69, 62, 73), um an einem ersten Ende des Spritzenkörpers (19) einen abnehmbaren Zusammenbau des Kolbens an dem Körper zu erlauben und um an einem zweiten, dem ersten entgegengesetzten Ende die abnehmbare Verbindung dieses selben Körpers mit einem Ergänzungsmittel (17) für die Einführung des Inhaltes der Spritze zu dem Körper des Patienten hin zu erlauben, **dadurch gekennzeichnet**, daß sie ferner aufweist:
- einen zweiten Kolben (49, 60), wobei die Mittel für den Zusammenbau zwischen dem ersten Kolben (25, 58) und dem Spritzenkörper (19) beim Zusammenbau untereinander nur an dem ersten Ende des Körpers kompatibel sind, ebenso für den zweiten Kolben (49,60) an dem zweiten Ende desselben Körpers, wo die Mittel für den Zusammenbau zwischen dem Körper und dem zweiten Kolben entsprechend gestaltet sind, wobei die an dem ersten Ende des Körpers für den Zusammenbau gebildeten Mittel ferner mit einer Verbindung kompatibel sind, die an dem Ergänzungsmittel (17) abnehmbar ist, außer der Anwesenheit des ersten Kolbens.

2. Vorrichtung nach Anspruch 1, **dadurch gekennzeichnet**, daß jeder Kolben (25, 49, 58, 60) einen Ring (35, 40) aufweist, der durch die Kolbenstange (21) durchquert wird und mit ersten Mitteln für den Zusammenbau (33, 41) versehen ist, die geeignet sind, mit zweiten Ergänzungsmitteln für den Zusammenbau (29, 31) des Spritzenkörpers zusammenzuwirken, um die vorgenannten Mittel für den Zusammenbau zu bilden, wobei die ersten Mittel für den Zusammenbau des einen der Kolben von denen des anderen Kolbens unterschiedlich sind.

3. Vorrichtung nach Anspruch 1 oder nach Anspruch 2, **dadurch gekennzeichnet**, daß der Spritzenkörper (19) in der Nähe der Mittel für den Zusammenbau (29, 31), welche er trägt, an seinen beiden Enden verschieden gebildet ist.

4. Vorrichtung nach Anspruch 3, **dadurch gekennzeichnet**, daß der Spritzenkörper (19) an jedem seiner zwei entgegengesetzten Enden (19a, 19b) ein Ansatzstück (29, 31) aufweist, welches die Mittel für den Zusammenbau des Körpers trägt, wobei der Körper nur an einem seiner zwei Enden ein rohrförmiges, kegelstumpfförmiges Teil (37) aufweist, das eines der Ansatzstücke durchquert, um den Zusammenbau an diesem Ende des Körpers nur mit dem entsprechenden Kolben (25, 49; 58, 60) zu erlauben.

5. Vorrichtung nach einem der Ansprüche 2 bis 4, **dadurch gekennzeichnet**, daß die zweiten Mittel für den Zusammenbau (29, 31), die auf dem Spritzenkörper (19) an jedem seiner Enden vorgesehen sind, gebildet sind, um ebenfalls abnehmbare Verbindungsmittel mit dem Ergänzungsmittel (17) für die Einführung seines Inhaltes zu dem Körper des Patienten hin bilden zu können.

6. Vorrichtung nach einem der vorhergehenden Ansprüche, **dadurch gekennzeichnet**, daß die Mittel für den Zusammenbau (29, 31) des Spritzenkörpers aus Gewinden für eine Verriegelung durch Verschrauben mit dem einen oder dem anderen der Kolben (35, 40; 58, 60) und/oder mit dem Ergänzungsmittel (17) für die Einführung des Inhaltes der Spritzen zu dem Körper des Patienten hin bestehen.

7. Vorrichtung nach einem der vorhergehenden Ansprüche, **dadurch gekennzeichnet**, daß die Kolben (25, 49; 58, 60) und der Spritzenkörper (19) äußere Sichtelemente (59, 61, 63, 65, 53, 55, 57) für das Markieren der für den Zusammenbau vorgesehenen Seite des Kolbens, die dem Spritzenkörper entspricht, aufweisen.

8. Vorrichtung nach Anspruch 7, **dadurch gekennzeichnet**, daß die äußeren Sichtelemente einen unterschiedlichen Farbencode für jeden Kolben aufweisen, der sowohl einem Teil des entsprechenden Kolbens und des Spritzenkörpers gemeinsam ist.

9. Vorrichtung nach einem der vorhergehenden Ansprüche, **dadurch gekennzeichnet**, daß an einem der Enden des Spritzenkörpers (19) die Mittel für den Zusammenbau (29; 66, 67) des letzteren mit denen des ersten Kolbens (49, 58) durch teilweises Bedecken des ersten Endes des Körpers durch ein Teil des ersten Kolbens zusammenwirken, während an dem zweiten Ende der zweite Kolben (25, 60) und der Körper in Zusammenbau nur durch teilweises in Eingriffkommen des Kolbens in dieses Ende des Spritzenkörpers zusammenwirken.

10. Vorrichtung nach einem der vorhergehenden Ansprüche, **dadurch gekennzeichnet**, daß der Inhalt des Spritzenkörpers (19) ein Blutfilter (1) ist, der in einem Gefäß implantierbar ist, um dort Blutkoagulate festzuhalten.
